# EUROPEAN PATENT SPECIFICATION

(11) **EP 4 048 288 B1**
(45) Date of publication and mention of the grant of the patent: **08.07.2026**
(21) Application number: 20883760.9
(22) Date of filing: 26.10.2020
(51) Int. Cl.: A61K 31/712, A61P 27/02, A61K 31/7088, A61K 9/00, A61K 31/713, A61K 47/60, C12N 15/115

(54) **ANTI-C5 AGENT FOR TREATMENT OF DRY AGE-RELATED MACULAR DEGENERATION (AMD) OR GEOGRAPHIC ATROPHY SECONDARY TO DRY AMD**
ANTI-C5-MITTEL ZUR BEHANDLUNG DER TROCKENEN ALTERSBEDINGTEN MAKULADEGENERATION (AMD) ODER DER GEOGRAFISCHEN ATROPHIE ALS SEKUNDÄRFOLGE DER TROCKENEN AMD
AGENT ANTI-C5 POUR LE TRAITEMENT DE LA DÉGÉNÉRESCENCE MACULAIRE LIÉE À L'ÂGE (DMLA) SÈCHE OU D'UNE ATROPHIE GÉOGRAPHIQUE SECONDAIRE À LA DMLA SÈCHE

(30) Priority: 27.10.2019 US 201962926558 P; 12.01.2020 US 202062960133 P
(43) Date of publication of application: 31.08.2022
(73) Proprietor: Astellas US LLC, Northbrook IL 60062 (US)
(72) Inventor: REZAEI, Kourous A., Chicago, IL 60601 (US)
(74) Representative: Longland, Emma Louise
(86) International application number: PCT/US2020/057422
(87) International publication number: WO 2021/091718

(56) References cited:
- WO-A1-2019/040397
- WO-A1-2019/040397
- US-A1- 2009 269 356
- US-A1- 2016 038 589
- JAFFE GLENN J. ET AL: "C5 Inhibitor Avacincaptad Pegol for Geographic Atrophy Due to Age-Related Macular Degeneration", OPHTHALMOLOGY, vol. 128, no. 4, 1 September 2020 (2020-09-01), AMSTERDAM, NL, pages 576 - 586, XP093092863, ISSN: 0161-6420, DOI: 10.1016/j.ophtha.2020.08.027
- ROSENFELD, PJ ET AL.: "Emixustat Hydrochloride for Geographic Atrophy Secondary to Age-Related Macular Degeneration", OPHTHALMOLOGY, vol. 125, no. 10, 30 April 2018 (2018-04-30), pages 1556 - 1567, XP085480879, DOI: 10.1016/j.ophtha.2018.03.059
- F HOFFMAN-LA ROCHE LTD.,: "A Phase III, Multicenter, Randomized, Double-Masked, Sham-Controlled Study to Assess the Efficacy and Safety of Lampalizumab Administered Intravitreally to Patients with Geographic Atrophy Secondary to Age-Related Macular Degeneration", PROTOCOL GX29176, VERSION 6, 24 September 2015 (2015-09-24), pages 201PP, XP055823795, Retrieved from the Internet <URL:https://jamanetwork.com/journals/ophth/articlepdf/2680577/eoi180031supp1_prod.pdf> [retrieved on 20210114]

## Description

### FIELD OF THE INVENTION

This invention relates to methods and compositions useful in subjects with dry age-related macular degeneration or geographic atrophy secondary to dry age-related macular degeneration. The methods involve administration of an effective amount of an anti-C5 agent.

### BACKGROUND OF THE INVENTION

Age-related macular degeneration ("AMD") is a disease characterized by progressive degenerative abnormalities in the macula, a small area in the central portion of the retina. AMD is characteristically a disease of the elderly and is the leading cause of blindness in individuals >50 years of age in developed countries. In the United States, it is estimated that approximately 6% of individuals 65-74 years of age, and 20% of those older than 75 years of age, are affected with AMD. Because of increasing life expectancy in developed and developing countries, the elderly sector of the general population is expected to increase at the greatest rate in coming decades. In the absence of adequate prevention or treatment measures, the number of cases of AMD with visual loss is expected to grow in parallel with the aging population.

AMD is classified into one of two general subgroups; the non-neovascular ("dry") form of the disease ("dry AMD") and the neovascular form of the disease ("wet AMD"). Dry AMD is more prevalent, accounting for approximately 90% of all AMD cases. It is characterized by degeneration of the macula and, with continued progression over multiple years, may ultimately result in atrophy of the central retina associated with central vision loss. By contrast, wet AMD, although less prevalent, is more likely to cause sudden, often substantial, loss of central vision.

Dry AMD is a significant cause of moderate and severe loss of central vision and is bilateral in most patients. In dry AMD, thinning of the retinal pigment epithelial cells (RPE) in the macula develops, along with other age-related changes to the adjacent retinal tissue layers. Dry AMD is characterized by the presence of drusen (yellow crystalline deposits that develop within the macula) located under the RPE. When the condition is severe, dry AMD results in marked thinning and/or atrophy of the macula, resulting from the loss of the RPE and associated capillaries (choriocapillaris). This form of late stage dry AMD is associated with thinning and loss of function of the neural retinal located above the affected RPE. This collective phenotype in late stage dry AMD is termed geographic atrophy ("GA"). The progressive degeneration of light-sensitive photoreceptor cells in GA leads to severe visual loss in affected eyes. In addition, dry AMD can progress to the wet form of the disease.

WO 2019/040397 A1 discloses the use of the anti-C5 aptamer ARC1905 in the monthly treatment of geographic atrophy.

Although dry AMD is the most common form of the disease, currently no approved therapy exists. The absence of treatment options for dry AMD represents an area of urgent unmet medical need, and a major public health concern for the rapidly increasing elderly population.

### SUMMARY OF THE INVENTION

The invention is according to the appended claims. In the following, references to methods of treatment using a product are to be interpreted as the product for use in that treatment of the disease or condition.

Aspects of the present invention relate to methods of treating a subject with GA secondary to dry AMD.

The methods comprise administering to the subject an anti-C5 agent comprising a C5-specific aptamer, in which the aptamer has a sequence of fCmGfCfCGfCmGmGfUfCfUfCmAmGmGfCGfCfUmGmAm GfUfCfUmGmAmGfUfUfUAfCfCfU mGfCmG-3T (SEQ ID NO: 1), in which fC and fU = 2' fluoro nucleotides, mG and mA = 2'-OMe nucleotides, all other nucleotides are 2'-OH, and 3T indicates an inverted deoxythymidine.

### BRIEF DESCRIPTION OF THE DRAWINGS

Reference is made to the following detailed description, which sets forth illustrative embodiments and the accompanying drawings of which:
Figure 1 shows a graph depicting the mean change in GA lesion area in dry AMD patients measured at 24 weeks and treated with either a 0.3-mg or 1-mg dose of ARC1905 monthly from weeks 0 to 24, as set forth in Example 1.
Figure 2 shows a graph depicting the mean change in GA lesion area in dry AMD patients measured at 24 weeks and 48 weeks and treated with either a 0.3-mg or 1-mg dose of ARC1905 monthly from weeks 0 to 48, as set forth in Example 1.
Figure 3 shows a graph depicting the least squares mean change from baseline in square root GA lesion area, based on the MRM model, in dry AMD patients measured at 6 and 12 months and treated with a 2-mg dose of ARC1905 or Sham, as set forth in Example 2.
Figure 4 shows a graph depicting the least squares mean change from baseline in square root GA lesion area, based on the MRM model, in dry AMD patients measured at 6 and 12 months and treated with a 4-mg dose of ARC1905 or Sham, as set forth in Example 2.
Figure 5 shows a graph depicting the least squares mean change from baseline in nonsquare root GA lesion area, based on the MRM model, in dry AMD patients measured at 6 and 12 months and treated with a 2-mg dose of ARC1905 or Sham, as set forth in Example 2.
Figure 6 shows a graph depicting the least squares mean change from baseline in nonsquare root GA lesion area, based on the MRM model, in dry AMD patients measured at 6 and 12 months and treated with a 4-mg dose of ARC1905 or Sham, as set forth in Example 2.

### DETAILED DESCRIPTION

The present invention relates to methods and compositions useful for subjects with GA secondary to dry AMD.
The methods comprise administering an anti-C5 agent to subjects in need thereof.

### Anti-C5 Agents

The term "anti-C5 agent" refers to an agent that reduces, or inhibits, either partially or fully, the activity or production of a C5 complement protein or a variant thereof. An anti-C5 agent may directly or indirectly reduce or inhibit the activity or production of a C5 complement protein or variant thereof. An anti-C5 agent may reduce or inhibit the conversion of C5 complement protein into its component polypeptides C5a and C5b. Anti-C5 agents may also reduce or inhibit the activity or production of C5a and/or C5b.

In the present invention, the anti-C5 agent comprises a C5-specific aptamer, in which the aptamer comprises a nucleotide sequence of fCmGfCfCGfCmGmGfUfCfUfCmAmGmGfCGfCfUmGmAmGfUfCfUmGmAmGfUfUfUAfCfCfU mGfCmG-3T (SEQ ID NO: 1), in which fC and fU = 2' fluoro nucleotides, mG and mA = 2'-OMe nucleotides, all other nucleotides are 2'-OH, and 3T indicates an inverted deoxythymidine.

The aptamer is conjugated to a polyethylene glycol moiety (PEG) via a linker. The PEG moiety has a molecular weight of about 40 kDa. The PEG moiety is conjugated via a linker to the 5' end of the aptamer. The PEG moiety conjugated to the 5' end is a PEG moiety of about 40 kDa molecular weight. The about 40 kDa PEG moiety is a branched PEG moiety.

The aptamer comprises a compound, ARC1905, having the structure set forth below: 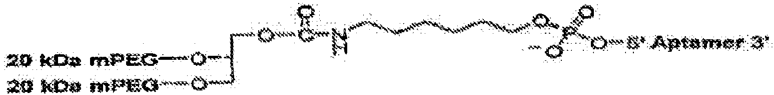 or a pharmaceutically acceptable salt thereof, where Aptamer = fCmGfCfCGfCmGmGfUfCfUfCmAmGmGfCGfCfUmGmAmGfUfCfUmGmAmGfUfUfUAfCfCfU mGfCmG-3T (SEQ ID NO: 1), in which fC and fU= 2'-fluoro nucleotides, mG and mA = 2'-OMe nucleotides, all other nucleotides are 2'-OH, and where 3T indicates an inverted deoxy thymidine. In some embodiments, each 20 kDa mPEG of the above structure has a molecular weight of about 20 kDa.

Examples of a pharmaceutically acceptable salt include, but are not limited to, sulfate, citrate, acetate, oxalate, chloride, bromide, iodide, nitrate, bisulfate, phosphate, acid phosphate, isonicotinate, lactate, salicylate, acid citrate, tartrate, oleate, tannate, pantothenate, bitartrate, ascorbate, succinate, maleate, gentisinate, fumarate, gluconate, glucaronate, saccharate, formate, benzoate, glutamate, methanesulfonate, ethanesulfonate, benzenesulfonate, p-toluenesulfonate, camphorsulfonate, pamoate, phenylacetate, trifluoroacetate, acrylate, chlorobenzoate, dinitrobenzoate, hydroxybenzoate, methoxybenzoate, methylbenzoate, o-acetoxybenzoate, naphthalene-2-benzoate, isobutyrate, phenylbutyrate, α-hydroxybutyrate, butyne-1,4-dicarboxylate, hexyne-1,4-dicarboxylate, caprate, caprylate, cinnamate, glycollate, heptanoate, hippurate, malate, hydroxymaleate, malonate, mandelate, mesylate, nicotinate, phthalate, teraphthalate, propiolate, propionate, phenylpropionate, sebacate, suberate, p-bromobenzenesulfonate, chlorobenzenesulfonate, ethylsulfonate, 2-hydroxyethylsulfonate, methylsulfonate, naphthalene-1-sulfonate, naphthalene-2-sulfonate, naphthalene-1,5-sulfonate, xylenesulfonate, and tartarate salts. The term "pharmaceutically acceptable salt" includes, but is not limited to, a hydrate of a compound of the invention and also may refer to a salt of an antagonist of the present invention having an acidic functional group, such as, but not limited to, a carboxylic acid functional group or a hydrogen phosphate functional group, and a base. Suitable bases include, but are not limited to, hydroxides of alkali metals such as sodium, potassium, and lithium; hydroxides of alkaline earth metal such as calcium and magnesium; hydroxides of other metals, such as aluminum and zinc; ammonia, and organic amines, such as unsubstituted or hydroxy-substituted mono-, di-, or tri-alkylamines, dicyclohexylamine; tributyl amine; pyridine; N-methyl, N-ethylamine; diethylamine; triethylamine; mono-, bis-, or tris-(2-OH-lower alkylamines), such as mono-, bis-, or tris-(2-hydroxyethyl)amine, 2-hydroxy-tert-butylamine, or tris-(hydroxymethyl)methylamine; N,N-di-lower alkyl-N-(hydroxyl-lower alkyl)-amines, such as N,N-dimethyl-N-(2-hydroxyethyl)amine or tri-(2-hydroxyethyl)amine; N-methyl-D-glucamine; and amino acids such as arginine, lysine, and the like.

### Methods of Use of the Anti-C5 Agents

The present invention relates to methods of treating a subject with GA secondary to dry AMD, comprising administering to the subject an anti-C5 agent as described herein. In some embodiments, administration of the anti-C5 agent may reduce, inhibit, slow, and/or reverse progression of GA. In some embodiments, administration of the anti-C5 agent may reduce, inhibit, slow, and/or reverse one or more symptoms of GA secondary to dry AMD, including, but not limited to, reduced or loss of central vision, loss of visual acuity, and reduced or inability to read. In some embodiments, administration of the anti-C5 agent may reduce, inhibit, slow, and/or reverse one or more of tissue- or cellular-level changes that is associated with GA secondary to dry AMD, including, but not limited to, growth of GA lesions, rate of growth or change in GA lesions, and the formation of drusen under the RPE.

In one aspect, the present invention relates to methods of reducing the rate of GA growth in a subject with GA secondary to dry AMD, comprising administering to the subject an anti-C5 agent as described herein. In some embodiments, administration of the anti-C5 agent to the subject may reduce the rate of GA growth by at least or about 5%, at least or about 10%, at least or about 15%, at least or about 20%, at least or about 25%, at least or about 30%, at least or about 35%, at least or about 40%, at least or about 45%, or at least or about 50%, as compared to that in a subject who is not administered the anti-C5 agent. Such a reduction of the rate of GA growth may be after a period of time of at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about 15 months, at least or about 18 months, or at least or about 24 months.

In one aspect, the present invention relates to methods of reducing the rate of change in GA area in a subject with GA secondary to dry AMD, comprising administering to the subject an anti-C5 agent as described herein. In some embodiments, administration of the anti-C5 agent to the subject may reduce the rate of change in GA area by at least or about 5%, at least or about 10%, at least or about 15%, at least or about 20%, at least or about 25%, at least or about 30%, at least or about 35%, at least or about 40%, at least or about 45%, or at least or about 50%, as compared to that in a subject who is not administered the anti-C5 agent. Such a reduction of the rate of change in GA area may be after a period of time of at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about 15 months, at least or about 18 months, or at least or about 24 months.

In one aspect, the present invention relates to methods of slowing or inhibiting the progression of GA in a subject, comprising administering to the subject an anti-C5 agent as described herein. In some embodiments, administration of the anti-C5 agent may slow or inhibit the progression of one or more symptoms of GA secondary to dry AMD including, but not limited to, reduced or loss of central vision, loss of visual acuity, and reduced or inability to read. In some embodiments, administration of the anti-C5 agent may slow or inhibit the progression of one or more of tissue- or cellular-level changes that are associated with GA secondary to dry AMD, including, but not limited to, growth of GA lesions, rate of growth or change in GA lesions, and the formation of drusen under the RPE.

In one aspect, the present invention relates to methods of slowing or inhibiting loss of visual acuity in a subject with GA secondary to dry AMD, comprising administering to the subject an anti-C5 agent as described herein. In some embodiments, administration of the anti-C5 agent to the subject may slow or inhibit the decrease in best corrected visual acuity (measured using ETDRS letters), as compared to that in a subject who is not administered the anti-C5 agent. Such a change may be measured between baseline and at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about l5 months, at least or about 18 months, or at least or about 24 months.

In one aspect, the present invention relates to methods of reversing loss of visual acuity in a subject with GA secondary to dry AMD, comprising administering to the subject an anti-C5 agent as described herein. In some embodiments, administration of the anti-C5 agent to the subject may increase the best corrected visual acuity (measured using ETDRS letters), as compared to that in a subject who is not administered the anti-C5 agent. Such an increase may be measured between baseline and at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about 15 months, at least or about 18 months, or at least or about 24 months.

In one aspect, the present invention relates to methods of slowing or inhibiting loss of low luminance visual acuity in a subject with GA secondary to dry AMD, comprising administering to the subject an anti-C5 agent as described herein. In some embodiments, administration of the anti-C5 agent to the subject may slow or inhibit the decrease in low luminance best corrected visual acuity (measured using ETDRS letters), as compared to that in a subject who is not administered the anti-C5 agent. Such a decrease may be measured between baseline and at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about l5 months, at least or about 18 months, or at least or about 24 months.

In one aspect, the present invention relates to methods of reversing loss of low luminance visual acuity in a subject with GA secondary to dry AMD, comprising administering to the subject an anti-C5 agent as described herein. In some embodiments, administration of the anti-C5 agent to the subject may increase the low luminance best corrected visual acuity (measured using ETDRS letters), as compared to that in a subject who is not administered the anti-C5 agent. Such an increase may be measured between baseline and at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about 15 months, at least or about 18 months, or at least or about 24 months.

In one aspect, the present invention relates to methods of reducing the formation of drusen in a subject, comprising administering to the subject an anti-C5 agent as described herein. In some embodiments, administration of the anti-C5 agent to the subject may reduce the formation of drusen as compared to that in a subject who is not administered the anti-C5 agent. A reduction in the formation of drusen may be determined by measuring how much drusen is formed under the retina after a period of time, such as after at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about 15 months, at least or about 18 months, or at least or about 24 months.

In one aspect, the present invention relates to methods of reducing the rate of formation of drusen in a subject, comprising administering to the subject an anti-C5 agent as described herein. In some embodiments, administration of the anti-C5 agent to the subject may reduce the rate of formation of drusen as compared to that in a subject who is not administered the anti-C5 agent. A reduction in the rate of formation of drusen may be determined by measuring how much drusen is formed under the retina over a period of time, such as after at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about 15 months, at least or about 18 months, or at least or about 24 months.

In one aspect, the present invention relates to methods of reversing the formation of drusen in a subject, comprising administering to the subject an anti-C5 agent as described herein. In some embodiments, administration of the anti-C5 agent to the subject may reverse the formation of drusen as compared to that in a subject who is not administered the anti-C5 agent. A reversal in the formation of drusen may be determined by measuring how much drusen is formed under the retina after a period of time, such as after at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about 15 months, at least or about 18 months, or at least or about 24 months.

In one aspect, the present invention relates to methods of reducing the amount of drusen in a subject, comprising administering to the subject an anti-C5 agent as described herein. In some embodiments, administration of the anti-C5 agent to the subject may reduce the amount of drusen as compared to that in a subject who is not administered the anti-C5 agent. A reversal in the formation of drusen may be determined by measuring how much drusen is present under the retina after a period of time, such as after at least or about 1 month, at least or about 2 months, at least or about 3 months, at least or about 4 months, at least or about 5 months, at least or about 6 months, at least or about 7 months, at least or about 8 months, at least or about 9 months, at least or about 10 months, at least or about 11 months, at least or about 12 months, at least or about 15 months, at least or about 18 months, or at least or about 24 months.

In some embodiments, the measurement using ETDRS letters may be as described in Early Treatment Diabetic Retinopathy Study Research Group (ETDRS), Manual of Operations, Baltimore: ETDRS Coordinating Center, University of Maryland. Available from: National Technical Information Service, 5285 Port Royal Road, Springfield, VA 22161; Accession No. PB8S 223006/AS; Ferris et al., Am J Ophthalmol 94:91-96, 1982; or Example 4, as described herein. In some embodiments, the vision testing uses one or more charts available from http://www.nei.nih.gov/photo/keyword.asp?conditions=Eye+Charts&match=all, e.g., ETDRS visual acuity Chart 1, 2 and/or R.

In some embodiments, the subject may be a mammal, which includes, but is not limited to, a human, monkey, cow, hog, sheep, horse, dog, cat, rabbit, rat, and mouse. In certain embodiments, the subject is a human.

In some embodiments, the subject may be treatment-naive, e.g., was not previously treated for the dry AMD, GA secondary to dry AMD, or GA secondary to AMD.

### Compositions for Therapeutic or Prophylactic Administration

The anti-C5 agent can be administered as a component of a composition that further comprises a pharmaceutically acceptable carrier or vehicle, e.g., a pharmaceutical composition. The anti-C5 agent, for example, can be admixed with a suitable carrier substance, and is generally present in an amount of 1-95% by weight of the total weight of the composition. The composition may be provided in a dosage form that is suitable for injection, in particular suitable for injection directly in the eye (e.g., intravitreal injection). The composition may be in form of, for example, suspensions, emulsions, or solutions.

Formulations for injection include sterile aqueous or non-aqueous solutions, suspensions, or emulsions. A variety of aqueous carriers can be used, e.g., water, buffered water, saline, and the like. Such formulations may also contain excipients such as preserving agents, wetting agents, buffering agents, emulsifying agents, dispersing agents, and suspending agents.

In some embodiments, excipients for compositions that comprise the anti-C5 agent include, but are not limited to, buffering agents, nonionic surfactants, preservatives, tonicity agents, sugars, amino acids, and pH-adjusting agents. Suitable buffering agents include, but are not limited to, monobasic sodium phosphate, dibasic sodium phosphate, and sodium acetate. Suitable nonionic surfactants include, but are not limited to, polyoxyethylene sorbitan fatty acid esters such as polysorbate 20 and polysorbate 80. Suitable preservatives include, but are not limited to, benzyl alcohol. Suitable tonicity agents include, but are not limited to sodium chloride, mannitol, and sorbitol. Suitable sugars include, but are not limited to, α,α-trehalose. Suitable amino acids include, but are not limited, to glycine and histidine. Suitable pH-adjusting agents include, but are not limited to, hydrochloric acid, acetic acid, and sodium hydroxide. In some embodiments, the pH-adjusting agent or agents are present in an amount effective to provide a pH of about 3 to about 8, about 6 to about 8, about 6.5 to about 8, about 4 to about 7, about 5 to about 6, about 6 to about 7, or about 7 to about 7.5. In certain embodiments, the pH-adjusting agent or agents are present in an amount effective to provide a pH of about 6.8 to about 7.8. In some embodiments, the compositions do not comprise a preservative. In some embodiments, the composition does not comprise an antimicrobial agent. In some embodiments, the composition does not comprise a bacteriostat.

In certain embodiments, the composition comprises sodium chloride, sodium phosphate monobasic (monohydrate), and sodium phosphate dibasic (heptahydrate), in which the pH of the composition is about 6.8 to about 7.8.

In some embodiments, the concentration of the anti-C5 agent in a composition is about 0.5 mg/mL to about 100 mg/mL. In some embodiments, the concentration of the anti-C5 agent in a composition is less than or about 100 mg/mL, less than about 50 mg/mL, less than about 40 mg/mL, less than about 30 mg/mL, less than about 25 mg/mL, less than about 20 mg/mL, less than about 15 mg/mL, less than about 10 mg/mL, or less than about 5 mg/mL. In certain embodiments, the concentration of the anti-C5 agent in a composition is about 0.3 mg/mL to about 100 mg/mL, about 0.3 mg/mL to about 50 mg/mL, about 1 mg/mL to about 50 mg/mL, about 5 mg/mL to about 40 mg/mL, about 10 to about 30 mg/mL, about 15 mg/mL to about 25 mg/mL, about 1 mg/mL, about 5 mg/mL, about 10 mg/mL, about 15 mg/mL, about 20 mg/mL, about 25 mg/mL, about 30 mg/mL, about 35 mg/mL, about 40 mg/mL, about 45 mg/mL, or about 50 mg/mL. In particular embodiments, the concentration of the anti-C5 agent in a composition is about 20 mg/mL.

### Administration and Dosage

The dosage of the anti-C5 agent is about 2 mg/eye. The anti-C5 agent is administered ocularly, by intravitreal injection.

The anti-C5 agent is administered in a dosing regimen comprising a loading phase and a maintenance phase.

The anti-C5 agent is administered in a dosing regimen comprising a loading phase that comprises a dose of about 2 mg/eye administered once a month for a duration of one year, followed by a maintenance phase that comprises a dose of about 2 mg/eye administered once every two months.

The amount of the composition comprising the anti-C5 agent administered to the subject can range from about 0.01 mL to about 0.2 mL administered per eye, or about 0.03 mL to about 0.15 mL administered per eye, or about 0.05 mL to about 0.10 mL administered per eye.

In some embodiments, the subject may receive more than one injection per eye of a lower dosage.

### Other Aspects of the Present Invention

The present invention relates to the anti-C5 agent as described herein for use in treating a subject with GA secondary to dry AMD.

The present invention relates to uses of the anti-C5 agent as described herein to treat a subject with GA secondary to dry AMD.

### EXAMPLES

### Example 1

A study was conducted in which 47 patients diagnosed with GA received treatment with three initial intravitreal injections of ARC1905 in dosages of either 0.3 mg/eye (24 patients) or 1 mg/ eye (23 patients), administered at Day 0, Week 4 and Week 8 with a follow up visit at Week 16. Patients received two subsequent injections at Week 24 and Week 36 followed by a final follow up visit at Week 48.

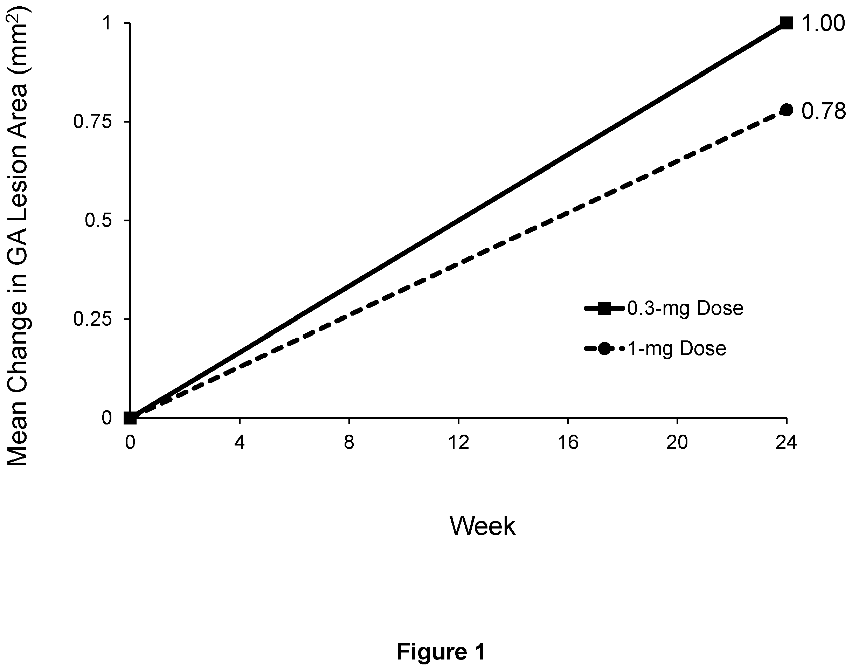

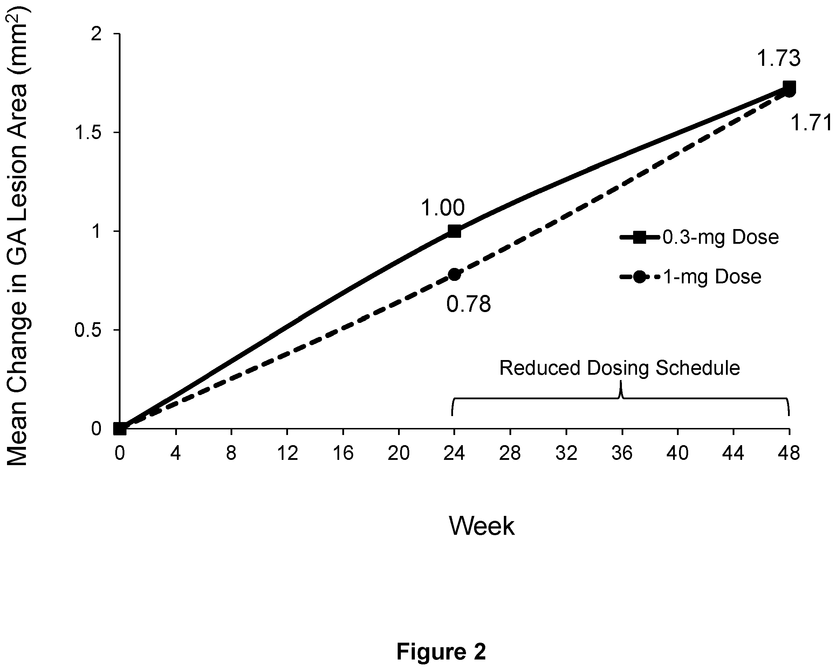

From Baseline to Week 24, the rate of growth of GA was slowed during the period of increased frequency of drug exposure (i.e. monthly dosing from day 0 to week 8). These results were dose related, as the change in GA lesion area from screening at Week 24 was 1.00 mm² in the 0.3 mg group and 0.78 mm² in the 1.0 mg group (see Figure 1). When drug exposure was reduced during the subsequent 24 weeks, the growth rates converged compared to baseline, as the change in GA lesion areas from screening at Week 48 was 1.73 mm² in the 0.3 mg group and 1.71 mm² in the 1.0 mg group (see Figure 2).

ARC1905 was well tolerated at both dose levels and there were no apparent differences between the dose groups. There were no adverse events considered to be related to ARC1905.

In summary, the results show a dose-dependent reduction in growth of the GA lesion from administration of ARC1905, indicating that ARC 1905 can slow the progression of GA in dry AMD patients.

### Example 2

A study was conducted to evaluate the safety and efficacy of ARC1905 intravitreous administration when administered in subjects with GA secondary to dry AMD.

The study consisted of two parts. In Part 1, 26 patients were randomized to receive monthly intravitreal injections of 1 mg of ARC1905; 25 patients were randomized to receive monthly intravitreal injections of 2 mg of ARC1905; and 26 patients were randomized to receive monthly sham injections. In Part 2 of the study, 83 patients were randomized to receive monthly intravitreal injections of 4 mg of ARC 1905, administered as two injections of 2 mg of ARC1905; 42 patients were randomized to receive monthly intravitreal injections of 2 mg of ARC1905 plus a sham injection; and 84 patients were randomized to receive monthly sham injections, administered as two separate sham injections.

For the study, ARC1905 was formulated at a concentration of 20 mg/mL (oligonucleotide mass) in phosphate buffered saline at pH 6.8-7.8 as a sterile aqueous solution, and presented in USP Type I 0.5 mL glass vials sealed with a halobutyl rubber stopper. For administering the 1-mg and 2-mg ARC1905 doses, formulations were prepared having a total injection volume of 0.05 mL and 0.1 mL, respectively. For administering the 4-mg ARC1905 dose, two injections of the 2-mg ARC1905 formulation were administered.

The statistical analysis of the data from the study used a mixed-effects repeated measures model (MRM model) to compare data for the ARC1905 2-mg and ARC1905 4-mg groups to the corresponding sham groups. The statistical analysis for the ARC 1905 2-mg group as compared to sham included a regression factor by part of the trial.

The primary efficacy endpoint of the study was the mean rate of change in GA over 12 months measured by fundus autofluorescence (FAF) based on readings at three time points (baseline, month 6, and month 12) and was calculated using the square root transformation of the GA area. The FAF images were assessed by an independent masked reading center. The results are shown in Table 1 below.

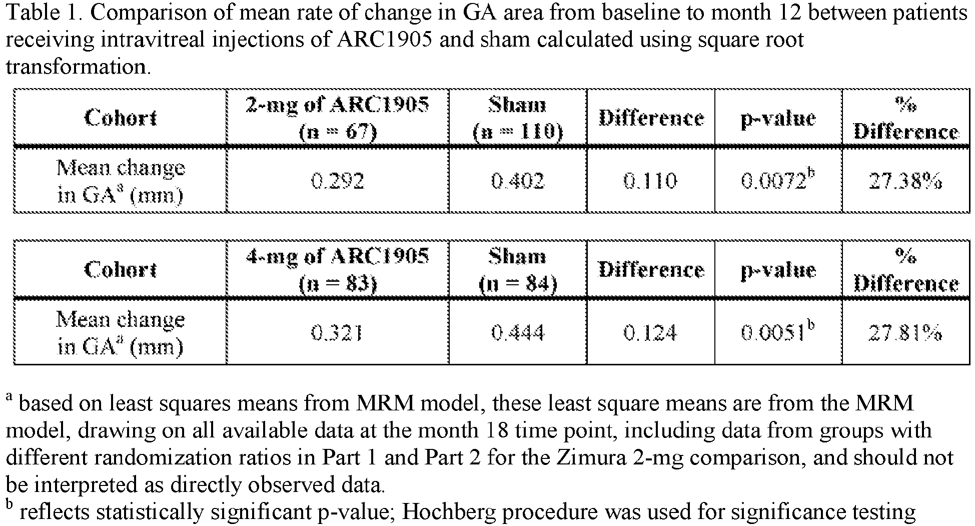

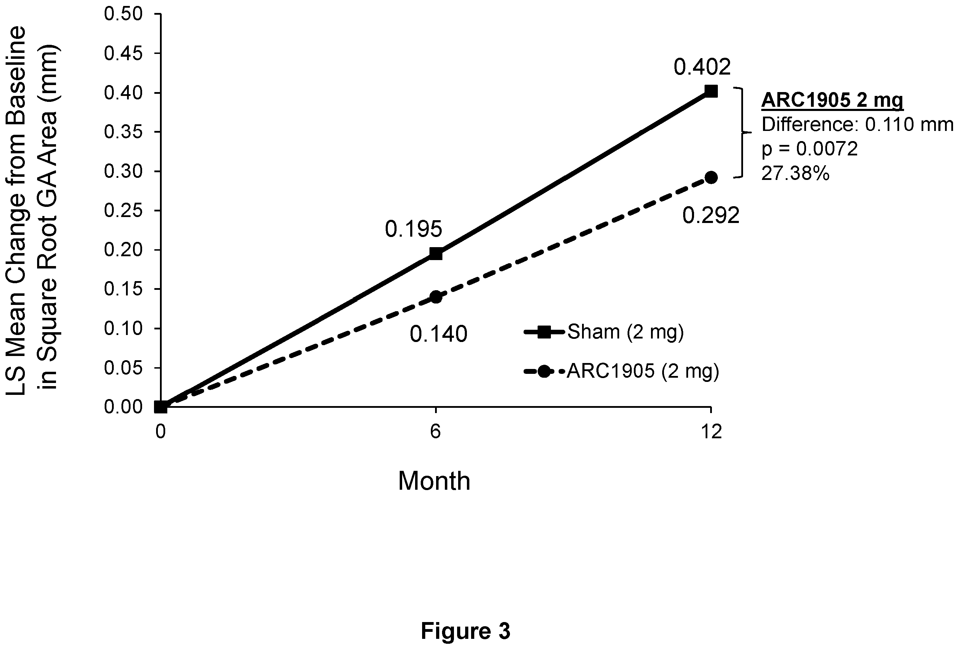

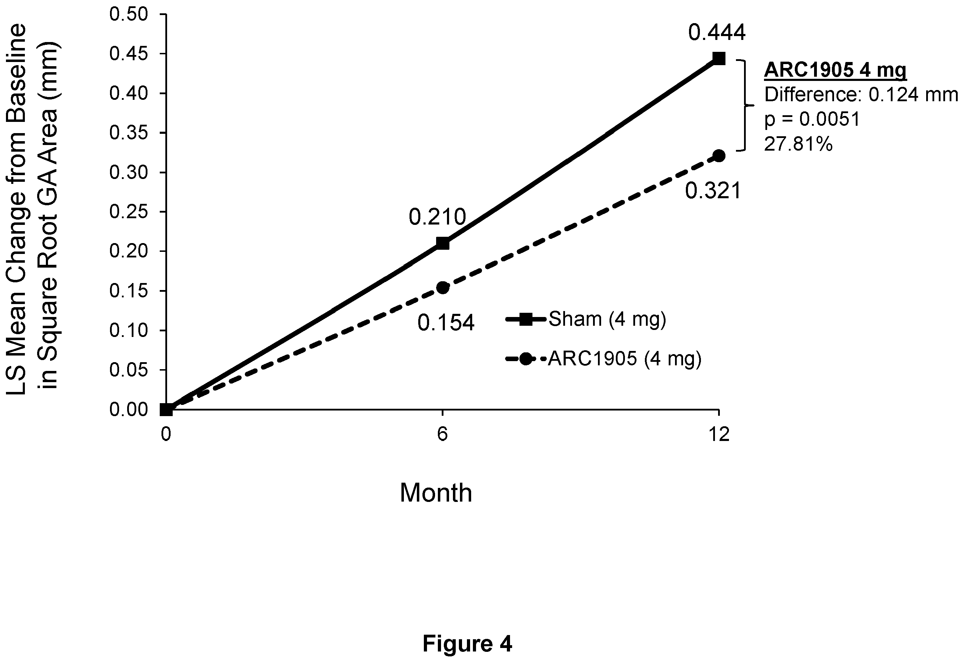

As shown in Table 1, the reduction in the mean rate of GA growth over 12 months was 27.38% (p-value = 0.0072) for the ARC1905 2-mg group as compared to the corresponding sham control group (see also Figure 3), and was 27.81% (p-value=0.0051) for the ARC1905 4-mg group as compared to the corresponding sham control group (see also Figure 4). These data for both dose groups were statistically significant.

The mean rate of change in GA growth over 18 months was measured by FAF, based on readings at four time points (baseline, month 6, month 12 and month 18) and was calculated using the square root transformation of the GA area. The FAF images were assessed by an independent masked reading center. The prespecified statistical analysis plan used a used a mixed-effects repeated measures model (MRM model) to compare data for the Zimura 2 mg and Zimura 4 mg groups to the corresponding sham groups. The results are shown in Table 2 below:

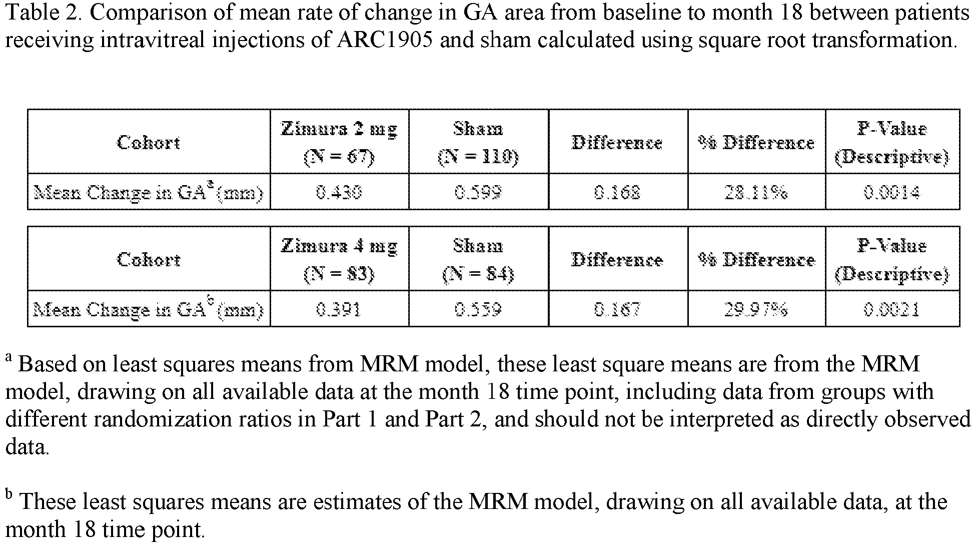

The analysis of the mean change in GA for ARC1905 2-mg group versus the Sham 2-mg group was adjusted for the fact that this dose of ARC1905 was tested in the two parts of the study, which had different randomization ratios. The least squares mean changes in GA in Part 1 and Part 2 are shown separately in Table 3.

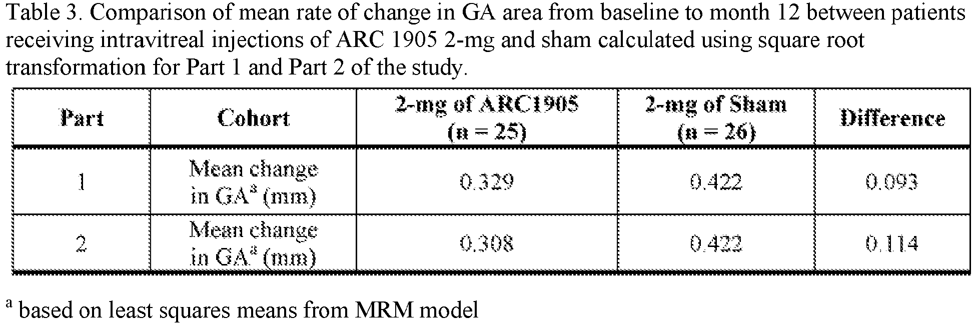

Consistent with the analysis performed at 12 months, the analysis of the mean change in GA growth for Zimura 2 mg as compared to the corresponding sham control group over 18 months was adjusted for the fact that this dose of Zimura was tested in both parts of the trial, each of which had different randomization ratios. The least squares mean changes in GA in Part 1 and Part 2 at month 18 are shown separately in Table 4:

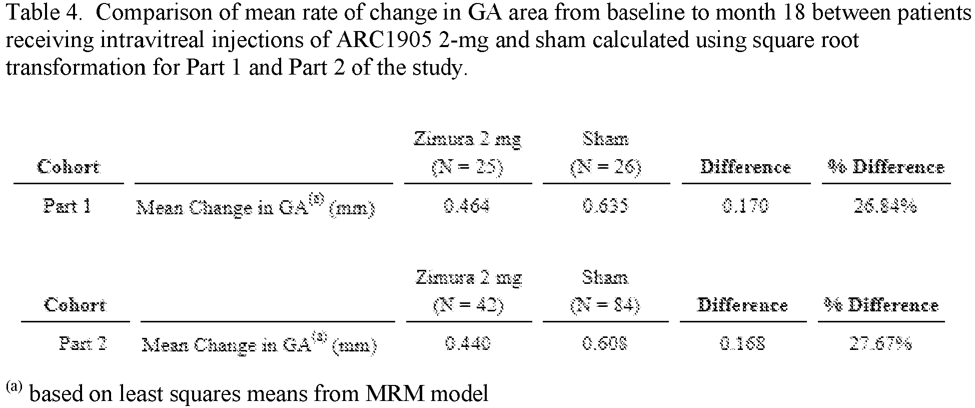

In addition, a preliminary, descriptive analysis indicated that, on average, there was a decrease in the percentage growth of GA from baseline to month 12 for patients receiving 1 mg of ARC1905 when compared to the corresponding sham control group. The overall data suggest a doseresponse relationship across treatment groups.

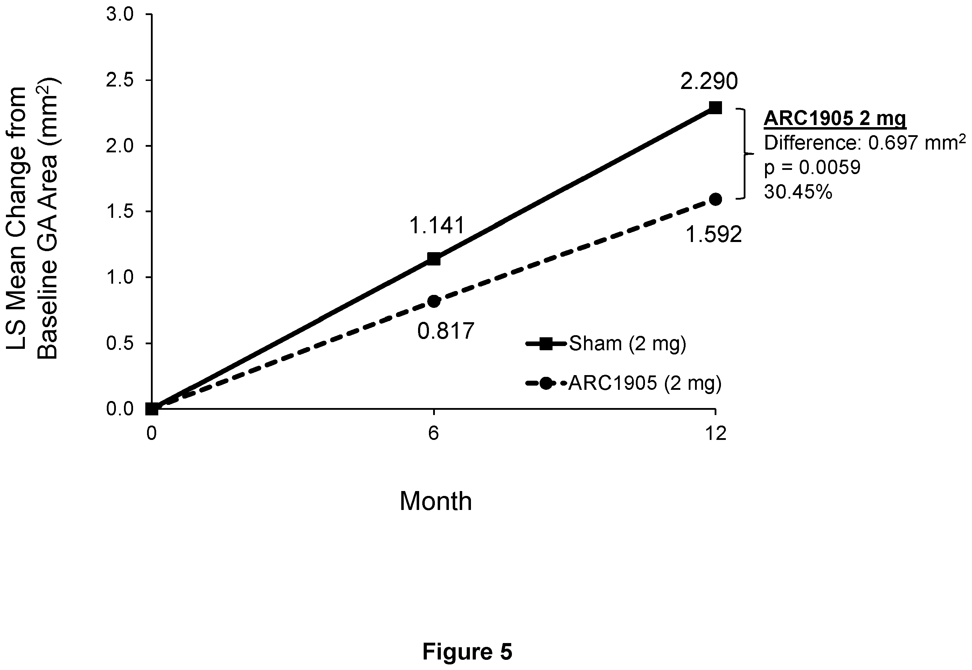

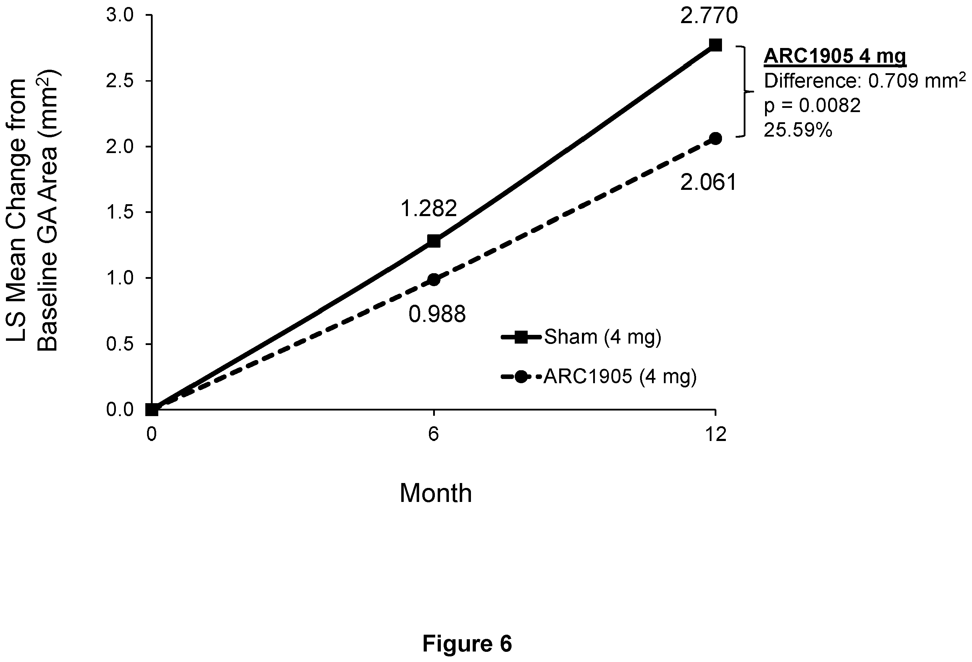

In addition to analyzing the mean rate of change in GA area using the square root transformation method (measured in millimeters (mm)), the mean rate of change in GA area was also analyzed using the observed GA areas (without the square root transformation, measured in square millimeters (mm²)), with the MRM model over 12 months. As shown in Table 5, the reduction in the mean rate of GA growth over 12 months was 30.45% (p-value = 0.0059) for the ARC 1905 2-mg group as compared to the corresponding sham control group (see also Figure 5), and was 25.59% (p-value = 0.0082) for the ARC1905 4-mg group as compared to the corresponding sham control group (see also Figure 6). These data for both dose groups also were statistically significant.

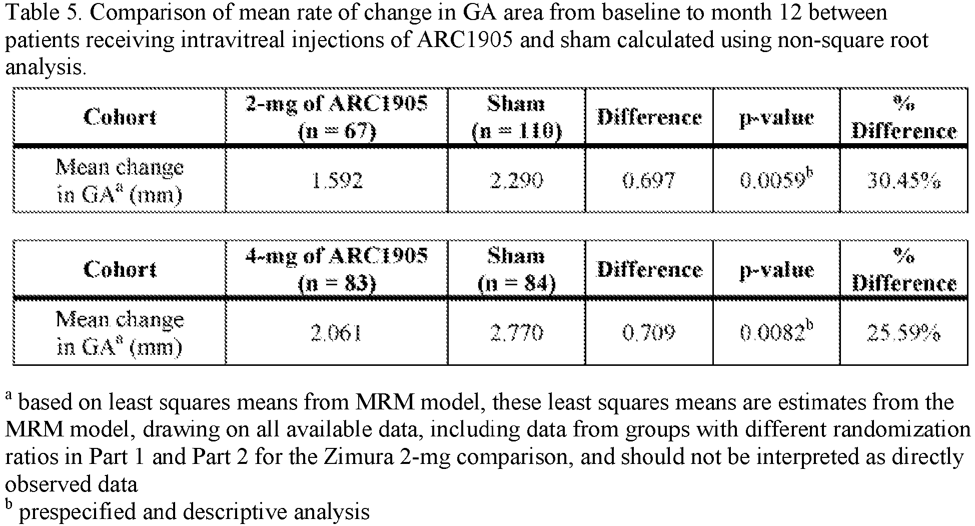

The mean rate of change in GA area was also analyzed over 18 months using the observed GA areas (without the square root transformation, measured in square millimeters (mm²)), with the MRM model. The observed mean GA area data for the Zimura 2 mg and Zimura 4 mg groups as compared to the corresponding sham control groups are summarized in Table 6:

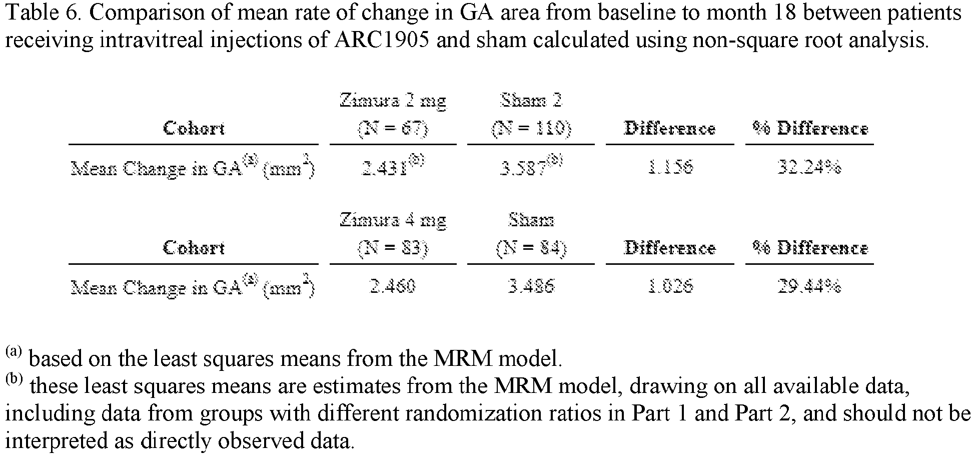

The second efficacy endpoints were the mean change in best corrected visual acuity (ETDRS letters) from baseline to month 12, the mean change in low luminance best corrected visual acuity (ETDRS letters) from baseline to month 12, the mean change in best corrected visual acuity (ETDRS letters) from baseline to month 18, and the mean change in low luminance best corrected visual acuity (ETDRS letters) from baseline to month 18. The results are shown in Tables 7, 8, 9, and 10 below.

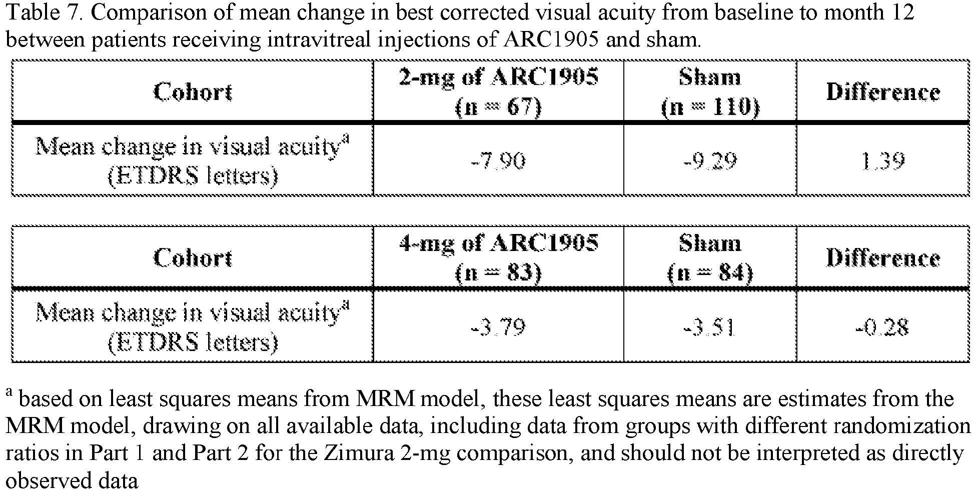

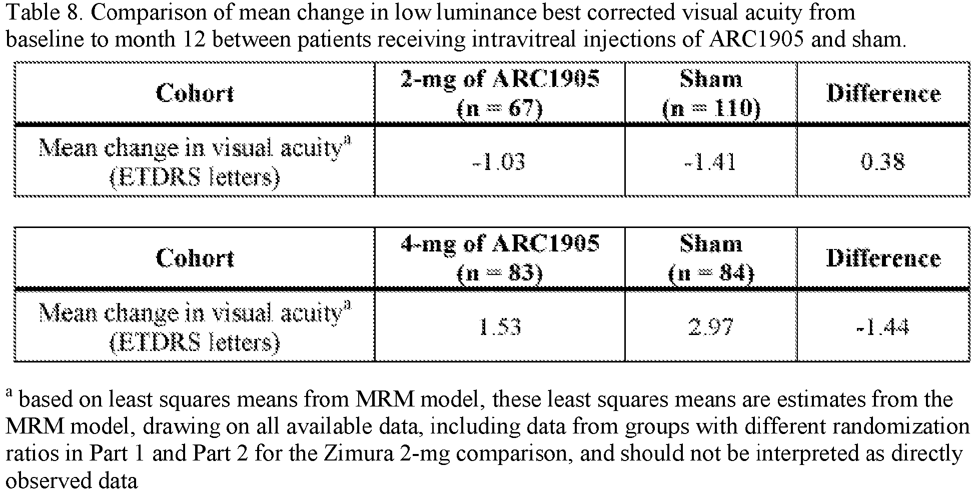

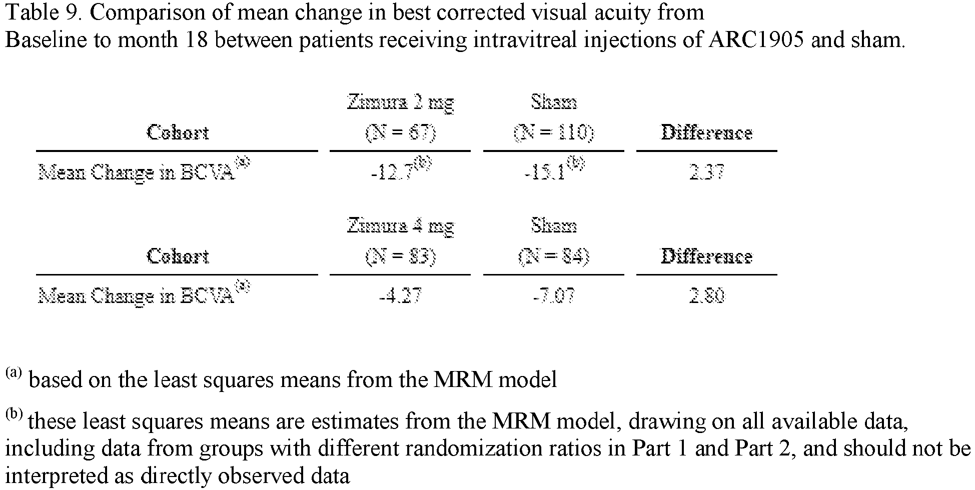

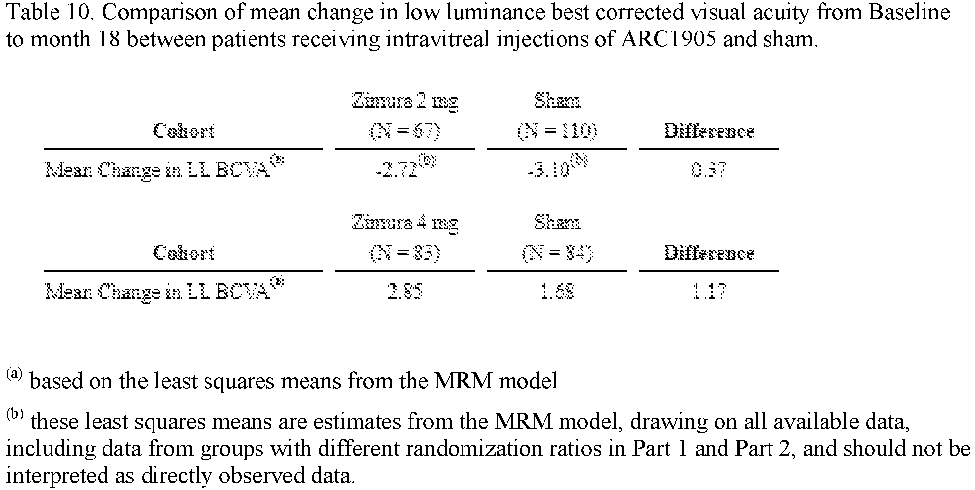

The safety endpoints of the study were: adverse events; vital signs (pulse, systolic and diastolic blood pressure); ophthalmic findings (intraocular pressure [IOP], ophthalmic examination, fluorescein angiogram, FAF, and Optical Coherence Tomography [OCT]); electrocardiogram (ECG) (12-lead); and laboratory variables (blood: hematology, renal function, hepatic function, and electrolytes; urinalysis).

ARC1905 was generally well tolerated after 12 months of administration. There was no ARC1905-related inflammation and there were no ARC1905-related discontinuations from the study. Further, there were no reports of ocular serious adverse events or of cases of endophthalmitis. During the 12 months, the incidence of choroidal neovascularization in the untreated fellow eyes was 10 patients (3.5%), and in the study eye was 3 patients (2.7%) in the sham control group, 6 patients (9.0%) in the ARC1905 2-mg group, and 8 patients (9.6%) in the ARC1905 4-mg group. The most frequently reported ocular adverse events were related to the injection procedure.

### Example 3

A study was conducted to evaluate the safety and efficacy of ARC1905 intravitreous administration when administered in subjects with GA secondary to dry AMD over a 24-month period.

Approximately 400 subjects are randomized at Day 1 in a 1:1 ratio to a monthly treatment group that is administered either (a) 2-mg of ARC1905 or (b) a sham dose. At Month 12, the subjects receiving monthly 2-mg of ARC1905 are re-randomized in a 1:1 ratio to receive either (i) 2-mg of ARC1905 administered monthly from Month 12 through Month 23, or (ii) a sham dose administered at Months 12, 14, 16, 18, 20, and 22, and 2-mg of ARC1905 administered every other month at Months 13, 15, 17, 19, 21, and 23. All subjects who were initially randomized to Sham (at Day 1) continue with monthly Sham injections through Month 23. All subjects have a final follow up visit at Month 24.

The primary efficacy endpoint of the study is the mean rate of change in GA over 12 months measured by FAF at three time points: Baseline, Month 6, and Month 12 (square root transformation). The safety endpoints are adverse events (AEs); vital signs (pulse, systolic and diastolic blood pressure); ophthalmic findings (best corrected visual acuity, low luminance best corrected visual acuity, intraocular pressure [IOP], and ophthalmic examination); electrocardiogram (ECG) (12-lead); and laboratory variables (blood: hematology, renal function, hepatic function, and electrolytes; urinalysis).

Subjects selected for the study is of either gender aged 50 years or greater and diagnosed with geographic atrophy secondary to dry AMD.

ARC1905 is formulated at a concentration of 20 mg/mL in phosphate buffered saline as a sterile aqueous solution and will be supplied in a sealed vial by the sponsor. It is preservative-free and intended for intravitreous injection only. The 2-mg dose has a total injection volume of 0.1 mL (100 µL).

Ophthalmic inclusion criteria for the study are as follows:
▪ Non-foveal GA secondary to dry AMD
▪ Total GA area 2 ≥ 2.5 and ≤ 17.5 mm² (1 and 7 disk areas [DA] respectively), determined by FAF screening images.
▪ If GA is multifocal, at least one focal lesion should measure ≥ 1.25 mm² (0.5 DA).
▪ GA in part within 1500 microns from the foveal center.
▪ The atrophic lesion must be able to be photographed in its entirety.
▪ Best corrected visual acuity in the study eye (SE) between 20/25 - 20/320, inclusive.
▪ Clear ocular media and adequate pupillary dilatation in both eyes to allow for all imaging procedures, including good quality stereoscopic fundus photography and fundus autofluorescence.
▪ Intraocular pressure of ≤ 21 mmHg or less in the SE.

General inclusion criteria for the study are as follows:
▪ Subjects of either gender aged ≥ 50 years.
▪ Women must be using two forms of effective contraception, be post-menopausal for at least 12 months prior to trial entry, or surgically sterile; if of child-bearing potential, a serum pregnancy test must be performed within 14 days prior to the first injection with a negative result. The two forms of effective contraception must be implemented during the trial and for at least 60 days following the last dose of test medication.
▪ Provide written informed consent.
▪ Ability to return for all trial visits for the 24-month duration of the study.

Ophthalmic exclusion criteria for the study are as follows:
▪ Evidence of CNV in either eye.
▪ GA secondary to any condition other than AMD in either eye (e.g., drug-induced).
▪ Any prior treatment for AMD (dry or wet) or any prior intravitreal treatment for any indication in either eye, except oral supplements of vitamins and minerals.
▪ Any ocular condition in the SE that would progress during the course of the study that could affect central vision or otherwise be a confounding factor.
▪ Concomitant treatment with any ocular or systemic medication that is known to be toxic to the lens, retina, or optic nerve.
▪ Presence of intraocular inflammation (≥ trace cell or flare), macular hole, pathologic myopia, epiretinal membrane, evidence of significant vitreo-macular traction, vitreous hemorrhage or aphakia (pseudophakia with or without an intact capsule is not an exclusion criteria).
▪ Presence or history of idiopathic or autoimmune-associated uveitis in either eye.
▪ Significant media opacities, including cataract, which might interfere with visual acuity, assessment of toxicity, fundus photography, or fundus autofluorescence.
▪ Subjects should not be entered if there is likelihood that they will require cataract surgery in the SE during the study.
▪ Presence of other causes of choroidal neovascularization, including pathologic myopia (spherical equivalent of -8 diopters or more, or axial length of 25 mm or more), ocular histoplasmosis syndrome, angioid streaks, choroidal rupture, and multifocal choroiditis.
▪ Any intraocular surgery or thermal laser within 3 months of trial entry. Any prior thermal laser in the macular region, regardless of indication.
▪ Any ocular or periocular infection (including blepharitis), or ocular surface inflammation in the past 12 weeks.
▪ History of any of the following procedures: Posterior vitrectomy, filtering surgery (e.g. trabeculectomy), glaucoma drainage device, comeal transplant, or retinal detachment.
▪ Any sign of diabetic retinopathy in either eye.

General exclusion criteria for the study are as follows:
▪ Any of the following underlying diseases including: history or evidence of severe cardiac disease (e.g., New York Heart Association [NYHA] Functional Class III or IV); history or clinical evidence of unstable angina, acute coronary syndrome, myocardial infarction, or revascularization within last 6 months; ventricular tachyarrhythmias requiring ongoing treatment; history or evidence of clinically significant peripheral vascular disease, such as intermittent claudication or prior amputation; clinically significant impaired renal (serum creatinine >2.5 mg/dL or status post renal transplant or receiving dialysis) or hepatic function; stroke (within 12 months of study entry); or any major surgical procedure within 1 month of trial entry.
▪ Previous therapeutic radiation in the region of the SE.
▪ Any treatment with an investigational agent in the past 60 days for any condition.
▪ Women who are pregnant or nursing.
▪ Known serious allergies to the fluoresce in dye used in angiography, povidone iodine, or to the components of the Zimura formulation.
▪ History of systemic treatment with any anti-complement agent in the past or the likelihood of treatment with any systemic anti-complement agent during the study.

## Claims

1. An anti-C5 agent for use in treating a subject with geographic atrophy secondary to dry age-related macular degeneration,
wherein the anti-C5 agent comprises a C5-specific aptamer comprising the following structure: 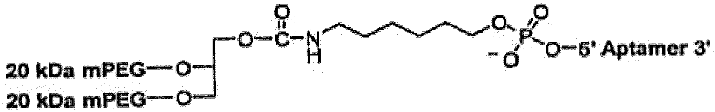
or a salt thereof,
wherein Aptamer = fCmGfCfCGfCmGmGfUfCfUfCmAmGmGfCGfCfUmGmAmGfUfC fUmGmAmGfUfUfUAfCfCfUmGfCmG-3T (SEQ ID NO: 1), wherein fC and fU = 2' fluoro nucleotides, mG and mA = 2' -OMe nucleotides, all other nucleotides are 2' -OH, and 3T indicates an inverted deoxythymidine,
wherein the anti-C5 agent is administered to the subject in a loading phase followed by a maintenance phase, wherein the loading phase comprises administering the anti-C5 agent at a dose of about 2 mg/eye for a duration of twelve months at a frequency in which the duration between doses is one month, and wherein the maintenance phase comprises administering the anti-C5 agent at a dose of about 2 mg/eye thereafter at a frequency in which the duration between doses is two months, and
wherein the anti-C5 agent is administered intraocularly to the subject via intravitreal injection into the eye.

2. An anti-C5 agent for use according to claim 1, wherein the administration of the anti-C5 agent to the subject slows or inhibits a decrease in low luminance best corrected visual acuity as compared to a subject who is not administered the anti-C5 agent.

3. An anti-C5 agent for use according to claim 1, wherein the administration of the anti-C5 agent to the subject increases the low luminance best corrected visual acuity as compared to a subject who is not administered the anti-C5 agent.

4. An anti-C5 agent for use according to claim 2, wherein the increase is measured between baseline and at least or about 1 month.

5. An anti-C5 agent for use according to claim 2, wherein the increase is measured between baseline and at least or about 6 months.

6. An anti-C5 agent for use according to claim 2, wherein the increase is measured between baseline and at least or about 8 months.

7. An anti-C5 agent for use according to claim 2, wherein the increase is measured between baseline and at least or about 12 months.

8. An anti-C5 agent for use according to claim 2, wherein the increase is measured between baseline and at least or about 18 months.

9. An anti-C5 agent for use according to claim 2, wherein the increase is measured between baseline and at least or about 24 months.

10. An anti-C5 agent for use according to claim 1, wherein the low luminance best corrected visual acuity is measured using ETDRS letters.

11. An anti-C5 agent for use according to claim 1, wherein the subject increases the low luminance best corrected visual acuity as compared to a subject who is not administered the anti-C5 agent.

12. An anti-C5 agent for use according to claim 1, wherein the subject is human.

## Patentansprüche

1. Anti-C5-Mittel zur Verwendung bei der Behandlung eines Subjekts mit geografischer Atrophie als Sekundärfolge trockener altersbedingter Makuladegeneration,
wobei das Anti-C5-Mittel ein CS-spezifisches Aptamer umfasst, das die folgende Struktur umfasst: 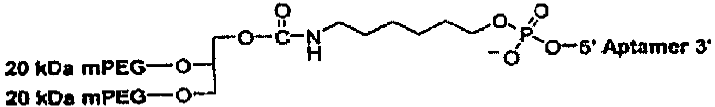
oder ein Salz davon,
wobei Aptamer =fCmGfCfCGfCmGmGfUfCfUfCmAmGmGfCGfCfUmGmAmGfUfCf UmGmAmGfUfUfUAfCfCfUmGfCmG-3T (SEQ ID NO: 1), wobei fC und fU = 2'-Fluor-Nukleotide, mG und mA = 2'-OMe-Nukleotide, alle anderen Nukleotide 2'-OH sind und 3T ein invertiertes Desoxythymidin angibt,
wobei das Anti-C5-Mittel dem Subjekt in einer Ladephase, gefolgt von einer Erhaltungsphase, verabreicht wird, wobei die Ladephase Verabreichen des Anti-C5-Mittels in einer Dosis von etwa 2 mg/Auge für eine Dauer von zwölf Monaten mit einer Häufigkeit umfasst, bei der die Dauer zwischen Dosen einen Monat beträgt, und wobei die Erhaltungsphase Verabreichen des Anti-C5-Mittels in einer Dosis von etwa 2 mg/Auge danach mit einer Häufigkeit umfasst, bei der die Dauer zwischen Dosen zwei Monate beträgt; und
wobei das Anti-C5-Mittel dem Subjekt intraokular über intravitreale Injektion in das Auge verabreicht wird.

2. Anti-C5-Mittel zur Verwendung nach Anspruch 1, wobei die Verabreichung des Anti-C5-Mittels an das Subjekt eine Abnahme der bestkorrigierten Sehschärfe unter geringer Leuchtdichte im Vergleich zu einem Subjekt, dem das Anti-C5-Mittel nicht verabreicht wird, verlangsamt oder hemmt.

3. Anti-C5-Mittel zur Verwendung nach Anspruch 1, wobei die Verabreichung des Anti-C5-Mittels an das Subjekt die bestkorrigierte Sehschärfe unter geringer Leuchtdichte im Vergleich zu einem Subjekt, dem das Anti-C5-Mittel nicht verabreicht wird, erhöht.

4. Anti-C5-Mittel zur Verwendung nach Anspruch 2, wobei die Erhöhung zwischen Ausgangswert und mindestens oder etwa 1 Monat gemessen wird.

5. Anti-C5-Mittel zur Verwendung nach Anspruch 2, wobei die Erhöhung zwischen Ausgangswert und mindestens oder etwa 6 Monaten gemessen wird.

6. Anti-C5-Mittel zur Verwendung nach Anspruch 2, wobei die Erhöhung zwischen Ausgangswert und mindestens oder etwa 8 Monaten gemessen wird.

7. Anti-C5-Mittel zur Verwendung nach Anspruch 2, wobei die Erhöhung zwischen Ausgangswert und mindestens oder etwa 12 Monaten gemessen wird.

8. Anti-C5-Mittel zur Verwendung nach Anspruch 2, wobei die Erhöhung zwischen Ausgangswert und mindestens oder etwa 18 Monaten gemessen wird.

9. Anti-C5-Mittel zur Verwendung nach Anspruch 2, wobei die Erhöhung zwischen Ausgangswert und mindestens oder etwa 24 Monaten gemessen wird.

10. Anti-C5-Mittel zur Verwendung nach Anspruch 1, wobei die bestkorrigierte Sehschärfe unter geringer Leuchtdichte unter Verwendung von ETDRS-Buchstaben gemessen wird.

11. Anti-C5-Mittel zur Verwendung nach Anspruch 1, wobei das Subjekt die bestkorrigierte Sehschärfe unter geringer Leuchtdichte im Vergleich zu einem Subjekt, dem das Anti-C5-Mittel nicht verabreicht wird, erhöht.

12. Anti-C5-Mittel zur Verwendung nach Anspruch 1, wobei das Subjekt ein Mensch ist.

## Revendications

1. Agent anti-C5 destiné à être utilisé dans le traitement d'un sujet atteint d'une atrophie géographique secondaire à une dégénérescence maculaire sèche liée à l'âge,
dans lequel l'agent anti-C5 comprend un aptamère spécifique de C5 comprenant la structure suivante : 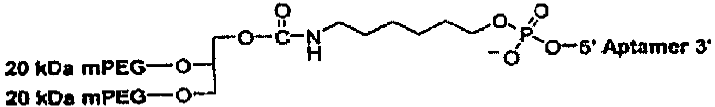
ou un sel de celui-ci,
dans lequel Aptamère = fCmGfCfCGfCmGmGfUfCfUfCmAmGmGfCGfCfUmGmAmGfUfCfUmGmAmGfUfUfUAfCf CfUmGfCmG-3T (SEQ ID N° : 1), où fC et fU = nucléotides 2'-fluoro, mG et mA = nucléotides 2'-OMe, tous les autres nucléotides sont 2'-OH, et 3T indique une désoxythymidine inversée,
dans lequel l'agent anti-C5 est administré au sujet dans une phase de charge suivie d'une phase d'entretien, dans lequel la phase de charge comprend l'administration de l'agent anti-C5 à une dose d'environ 2 mg/œil pendant une durée de douze mois à une fréquence telle que la durée entre les doses est d'un mois, et dans lequel la phase d'entretien comprend l'administration de l'agent anti-C5 à une dose d'environ 2 mg/œil par la suite à une fréquence telle que la durée entre les doses est de deux mois ; et
dans lequel l'agent anti-C5 est administré au sujet par voie intraoculaire par injection intravitréenne dans l'œil.

2. Agent anti-C5 destiné à être utilisé selon la revendication 1, dans lequel l'administration de l'agent anti-C5 au sujet ralentit ou inhibe une diminution de la meilleure acuité visuelle corrigée en faible luminance par comparaison avec un sujet auquel l'agent anti-C5 n'est pas administré.

3. Agent anti-C5 destiné à être utilisé selon la revendication 1, dans lequel l'administration de l'agent anti-C5 au sujet augmente la meilleure acuité visuelle corrigée en faible luminance par comparaison avec un sujet auquel l'agent anti-C5 n'est pas administré.

4. Agent anti-C5 destiné à être utilisé selon la revendication 2, dans lequel l'augmentation est mesurée entre la valeur initiale et au moins ou environ 1 mois.

5. Agent anti-C5 destiné à être utilisé selon la revendication 2, dans lequel l'augmentation est mesurée entre la valeur initiale et au moins ou environ 6 mois.

6. Agent anti-C5 destiné à être utilisé selon la revendication 2, dans lequel l'augmentation est mesurée entre la valeur initiale et au moins ou environ 8 mois.

7. Agent anti-C5 destiné à être utilisé selon la revendication 2, dans lequel l'augmentation est mesurée entre la valeur initiale et au moins ou environ 12 mois.

8. Agent anti-C5 destiné à être utilisé selon la revendication 2, dans lequel l'augmentation est mesurée entre la valeur initiale et au moins ou environ 18 mois.

9. Agent anti-C5 destiné à être utilisé selon la revendication 2, dans lequel l'augmentation est mesurée entre la valeur initiale et au moins ou environ 24 mois.

10. Agent anti-C5 destiné à être utilisé selon la revendication 1, dans lequel la meilleure acuité visuelle corrigée en faible luminance est mesurée à l'aide de lettres ETDRS.

11. Agent anti-C5 destiné à être utilisé selon la revendication 1, dans lequel le sujet présente une augmentation de la meilleure acuité visuelle corrigée en faible luminance par comparaison avec un sujet auquel l'agent anti-C5 n'est pas administré.

12. Agent anti-C5 destiné à être utilisé selon la revendication 1, dans lequel le sujet est humain.
